(19) Europäisches Patentamt
European Patent Office
Office européen des brevets

(11) **EP 2 163 887 B1**

(12) **EUROPÄISCHE PATENTSCHRIFT**

(45) Veröffentlichungstag und Bekanntmachung des Hinweises auf die Patenterteilung:
**24.12.2014   Patentblatt 2014/52**

(51) Int Cl.:
*G01N 27/02* (2006.01)          *G01N 33/28* (2006.01)
*G01N 33/30* (2006.01)

(21) Anmeldenummer: **09000244.5**

(22) Anmeldetag: **09.01.2009**

(54) **Verfahren und Schaltungsanordnung zur Messung physikalischer Grössen in Fluiden sowie deren Verwendung**

Method and circuit assembly for measuring physical parameters in fluids and their application

Procédé et circuiterie destinés à la mesure de grandeurs physiques dans des fluides et leur utilisation

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **15.09.2008   DE 102008047366**

(43) Veröffentlichungstag der Anmeldung:
**17.03.2010   Patentblatt 2010/11**

(73) Patentinhaber:
• **Kuipers, Ulrich**
  **57462 Olpe (DE)**
• **Mauntz, Manfred**
  **65760 Eschborn (DE)**

(72) Erfinder:
• **Kuipers, Ulrich**
  **57462 Olpe (DE)**

• **Mauntz, Manfred**
  **65760 Eschborn (DE)**

(74) Vertreter: **Köchling, Conrad-Joachim**
  **Patentanwälte Köchling, Döring PartG mbB**
  **Fleyer Strasse 135**
  **58097 Hagen (DE)**

(56) Entgegenhaltungen:
**EP-A- 1 122 886          EP-A- 1 424 562**
**WO-A-01/09625          WO-A-01/75459**
**DE-A1- 3 517 065          DE-A1- 19 628 690**
**DE-A1- 19 819 013          US-A- 5 231 358**
**US-A- 5 889 200          US-A- 6 028 433**
**US-A1- 2006 105 467**

EP 2 163 887 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein Verfahren und eine Schaltungsanordnung zur Messung der Qualitätsveränderung von zur Schmierung oder zur Isolierung eingesetzten Ölen, von Parametern von Erdölen und deren Beiprodukte und zur Messung von Impedanzkomponenten elektrisch gering oder elektrisch nicht leitender Fluide, dessen Realisierung und dessen Anwendung. Insbesondere werden die spezifische elektrische Leitfähigkeit und die Dielektrizitätszahl gemessen. Wegen deren Temperaturabhängigkeiten wird zusätzlich die Öltemperatur gemessen. Sowohl Abriebkomponenten, zerrissene Ölmoleküle, Verschmutzungen, wie mechanische Verschmutzungen, Salze, Säuren, Wasser, sich bildende Ölseifen usw. bewirken eine Erhöhung der elektrischen Leitfähigkeit. Basierend auf der Tatsache, dass die elektrische Leitfähigkeit von Öl außerordentlich gering gegenüber der Leitfähigkeit der Verschmutzungsprodukte ist, besteht ein direkter Zusammenhang zwischen der elektrischen Leitfähigkeit und dem verschmutzungsgrad.

Die Änderung des Verschmutzungsgrades zeigt wiederum den aktuellen Verschleiß oder den Verschmutzungsgrad bzw. die Zusammensetzung von Erdölen und deren Beiprodukte. Beispiele für Anwendungsbereiche sind die Überwachung von Getriebeölen, Hydraulikölen, Transformatoren, Motorölen, Überwachung bei der Erdölförderung und -aufbereitung, usw.

Konzeptionell vergleichbare Messungen haben sich bei der Leitfähigkeitsmessung von Wasser etabliert. Dabei sind die elektrischen Leitfähigkeiten allerdings mit um viele Zehnerpotenzen größeren und daher sehr viel einfacher zu messen. Auch bei der Wassermessung ist die elektrische Leitfähigkeit ein Summenparameter für die Ionenkonzentration und/oder Verschmutzungen. Allerdings wird mit dem erfindungsgemäßen Sensorsystem zusätzlich die Dielektrizitätszahl vorzugsweise zur Feuchtemessung, Wassergehaltsbestimmung, zur Erfassung des Abbaus von Additiven und/oder zur Messung von Gasanteilen gemessen.

Der aktuelle Stand der Technik ist insbesondere in der Patentschrift DE 196 28 690 wiedergegeben. Die darin beschriebenen Verfahren zur Messung der elektrischen Leitfähigkeit und der Dielektrizitätszahl über getaktete Gleichspannungen oder getaktete Gleichströme als Messsignale und den in der Patentschrift beschriebenen Merkmalen haben sich in der Anwendung als außerordentlich vorteilhaft erwiesen. Ein Nachteil des elektronischen Verfahrens ist jedoch, dass "die Integration bei jeder Änderung des Testsignals (Flanken) auf null gesetzt wird". Auch beim Öffnen des Analogschalters entsteht eine Ladungsinjektion (Charge Injection), die das Messergebnis verfälscht. Zur Messung der Leitfähigkeit und der Dielektrizitätszahl werden zwei Sensoren benötigt, oder es muss die Sensorelektronik umgeschaltet werden. Zudem ist die Sensorelektronik relativ komplex und sie beinhaltet weitere Fehlerquellen. Die mechanische Realisierung des Basissensors ist ebenfalls sehr aufwendig. Ein weiterer Nachteil ist, dass mit dem beschriebenen Sensorkonzept die zu messenden Ölparameter und damit letztlich auch die Ölqualität nicht im eigentlichen Schmierspalt gemessen werden können.

Die Patentanmeldung Nr. DE 10217383 der Siemens AG zum Thema "Zylinderkopfintegriertes Dieseleinspritzsystem mit Ölsensor" behandelt Injektoren, die vollständig innerhalb des Zylinderkopfes einer Brennkraftmaschine integriert sind. In der Anmeldeschrift ist ein Ölsensor zur Erfassung der Qualität des Motoröls und des Ölfüllstands vorgesehen. Zudem wird ausgeführt: "Vorzugsweise erfasst der Ölsensor die Qualität und/oder die Füllstandshöhe des Motoröls anhand einer Dielektrizitätskonstante des Motoröls." Das Patent DE 199 63 204 der Firma Cummins Inc. Columbus, ind., US, beinhaltet ein Verfahren zum Bestimmen der Restlebensdauer und/oder Restnutzungsdauer von Motoröl in einem Motor, wobei Motorparameter wie die Russkonzentration und/oder Viskosität des Motoröls gemessen werden.

Die in den zitierten Patenten vorgeschlagenen Lösungen haben die Nachteile von aufwendigen Basissensoren und Sensorelektroniken. Zudem ist deren Messgenauigkeit nicht optimal.

Der Erfindung liegt die Aufgabe der Realisierung von hochgenauen und dennoch einfach und kostengünstig realisierbaren Basissensoren und Sensorelektroniken für Messungen der Qualitätsveränderungen von Ölen, Maschinen, Transformatoren, Turbinen, Kompressoren, Motoren usw. und deren Anwendungen insbesondere zur Indizierung von vorbeugenden Wartungsmaßnahmen, unzulässigen Betriebszuständen, hohen Belastungen usw. bereits lange vor einer vorbeugenden Instandsetzung, sowie zur Qualitätsbewertung und zum Zustandsmonitoring zugrunde. Mit dem erfindungsgemäßen Sensorsystem soll auch unmittelbar im Schmierspalt gemessen werden können. Das erfindungsgemäße Ölsensorsystem ermöglicht eine zustandsorientierte, vorbeugende Wartung von Maschinen, Transformatoren, Turbinen, Kompressoren, Motoren usw. Der interdisziplinäre Ansatz einer quantifizierten Bewertung der Veränderung des Systems Öl-Getriebe, Öl-Motor usw. über die mit dem erfindungsgemäßen Sensorsystem gemessene momentane Ölverschmutzung und deren zeitlicher Veränderung, ermöglicht eine Bewertung des Getriebe-, Motor-, Transformatorenverschleißes usw. und des Ölverschleißes. Es können vorbeugende Wartungsmaßnahmen bereits lange vor einer vorbeugenden Instandsetzung indiziert werden.

Ein weiterer Einsatzbereich des erfindungsgemäßen Sensorsystems ist die Messung von geförderten Erdölen und deren Beiprodukte und/oder die Zusammensetzung von Erdölprodukten. Hierzu werden insbesondere Summenparameter von Salzen, Säuren, Ionenkonzentrationen, Wasseranteile und Gasen im Erdöl oder von Erdölprodukten gemessen.

Die elektrische Leitfähigkeit und die Dielektrizitätszahl des Öles werden unabhängig voneinander gemessen. Die Feinstverschmutzung der Öle wie z.B. Abrieb, zerschlagene Ölmoleküle, die Bildung von Seifen usw. schlagen sich insbesondere in einer Erhöhung der elektrischen Leitfähigkeit nieder. Zur Messung des Abbaus von Additiven und zur Bestimmung des Wassergehaltes wird zusätzlich die Dielektrizitätszahl der Öle gemessen. Zur Vermeidung von Polarisationseffekten werden als Messsignale mittelwertfreie Wechselgrößen genutzt. Die Erfindung beinhaltet auch die Entwicklung eines elektronischen Messverfahrens mit getakteten, mittelwertfreien Messsignalen zur getrennten Messung des Widerstandes und der Kapazität des Basissensors. Erfindungsgemäß werden der Basissensorwiderstand $R_x$ und die Basissensorkapazität $C_x$ aus einem Messsignal bestimmt. Hieraus werden die Leitfähigkeit und die Dielektrizitätszahl berechnet. Die elektrische Leitfähigkeit $\kappa$ und die Dielektrizitätszahl sind temperaturabhängig, wobei die Temperaturkoeffizienten weniger vom Öl, sondern insbesondere auch von den Ölverschmutzungen abhängig sind, zudem verändert sich die Sensorgeometrie in Abhängigkeit von der Temperatur. Eine selbstlernende adaptive Temperaturkompensation bzw. eine Umrechnung auf eine Referenztemperatur sind ebenfalls Bestandteile der Erfindung.

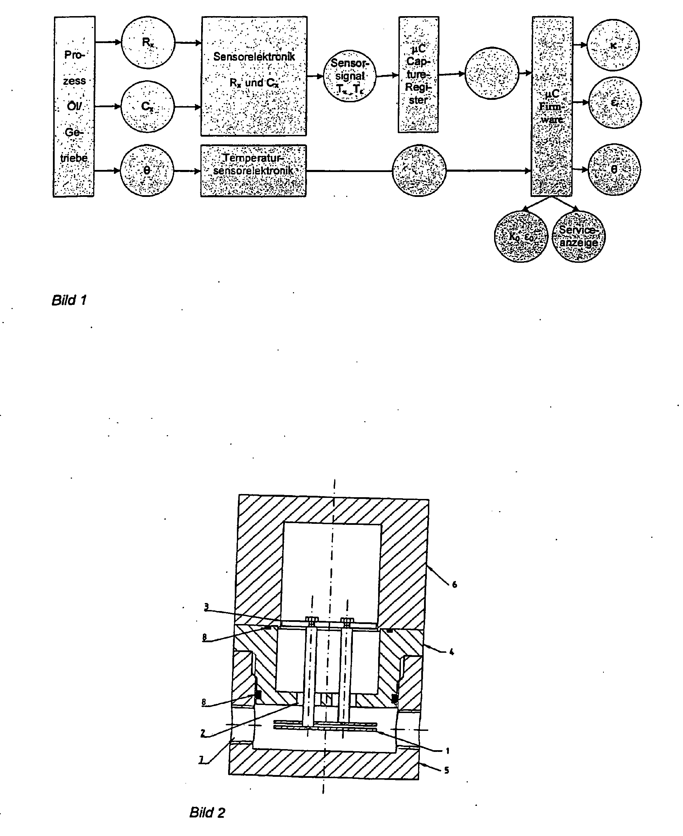

In Bild 1 ist ein Instanzennetz eines Ausführungsbeispiels des Ölsensorsystems gezeigt.

Instanzennetze bestehen aus rechteckig dargestellten Funktionseinheiten, den so genannten Instanzen, und kreisförmig dargestellten Datenräumen. Instanzen sind Funktionseinheiten mit Autonomie und definierten Zuständigkeiten. Sie sind durch Eingänge und rückkopplungsfreie Ausgänge charakterisiert.

Instanzen kommunizieren über die als Kreise dargestellten Datenräume miteinander. Datenzugriffe werden über Pfeile zwischen Datenräumen und Instanzen charakterisiert. Ein Instanzennetz veranschaulicht den Datenfluss durch ein System. Es erlaubt eine statische Interpretation des Einzugsbereichs der einzelnen Funktionseinheiten. Instanzen und Instanzennetze können stufenweise verfeinert werden. Beispiele für Instanzen sind Hardwarekomponenten, Softwareprogramme, Sensoren, Prozessoren, Automatisierungssysteme oder auch der Bediener eines Messsystems. Datenräume sind z.B.: Signale auf Leitungen, Arbeitsspeicher, Messsignale, Bildschirme oder Ausdrucke.

Eine erfindungsgemäße Basissensorkonstruktion wird nachfolgend beschrieben. Bild 2 zeigt eine Übersichtsskizze einer Konstruktionsvariante des realisierten Sensorsystems.

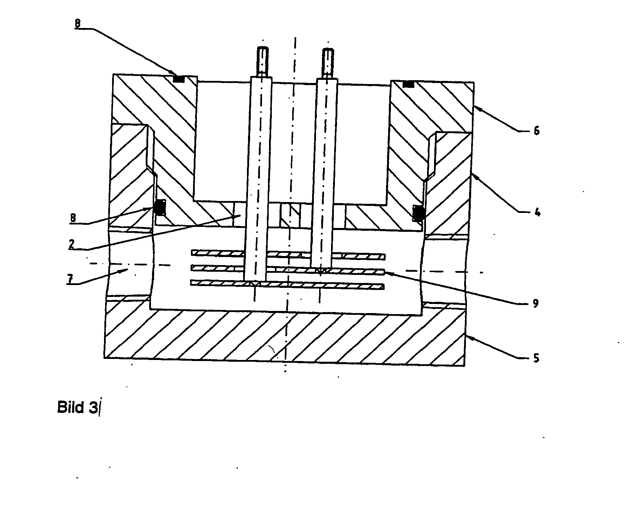

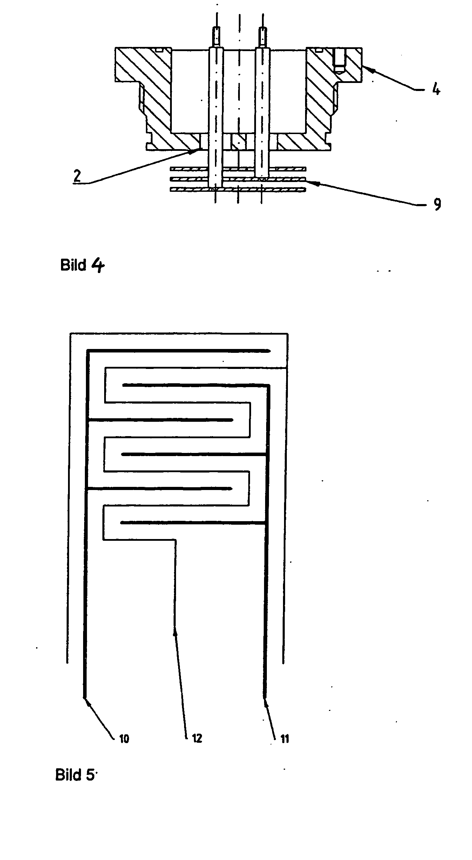

Der aktive Basissensorteil besteht hier aus zwei oder mehr Basissensorplatten 1 mit der Fläche A, die im konstanten Abstand 1 zueinander an den Metallstiften einer Glas/Metall-Durchführung 2 befestigt sind. Die Platten des Basissensors sind mittig in der Messkammer platziert, so dass eine günstige und richtungsunabhängige Anströmung des durchfließenden Mediums gewährleistet wird. Eine spezielle Ausrichtung der Sensorgehäuseteile ist bei dieser Konstruktion nicht erforderlich. Die Materialien des Sensorgehäuses und der Stifte der Glas/Metall-Durchführung 2 sind in ihren Ausdehnungseigenschaften genau an die Materialeigenschaften des verarbeitenden Glases der Durchführung angepasst. Bild 3 zeigt Glas/Metall-Durchführungen 2 im Sensorgehäuse, Bild 4 eine Basissensoranordnung mit drei Elektrodenplatten 9.

Die Leitfähigkeit elektrisch isolierender Konstruktionselemente und die Leitfähigkeit der Isolierung elektrischer Durchführungen liegen in gleichen Größenordnungen, wie die elektrische Leitfähigkeit der zu messenden reinen Öle. Die Leitfähigkeit der Durchführungen und Trägermaterialien dürfen aber nicht in das Messergebnis eingehen. Dieses Ziel wird in Verbindung mit der erfindungsgemäßen, präzisen Sensorelektronik erreicht. Hierzu wird auf eine Sensorelektrode das Testsignal, vorzugsweise eine abschnittsweise konstante Spannung oder ein abschnittsweise konstanter Strom geschaltet, die andere Elektrodenplatte wird erfindungsgemäß auf den invertierenden Eingang eines vorzugsweise auf virtueller Masse liegenden, stromgegengekoppelten Operationsverstärkers geschaltet. Alternativ ist die Schaltung auf den invertierenden Eingang eines spannungsgegengekoppelten Operationsverstärkers.

Eine alternative Ausgestaltung ist das unmittelbare Einschrauben einer Anordnung nach Bild 4 in eine Rohrwandung, z.B. eine Erdölpipeline.

Eine alternative Basissenorrealisierung ist eine planare Elektrodenanordnung. Sie wird vorzugsweise als eine Gold-Platin-Legierung auf Keramiksubstrat hergestellt. Das Herstellungsverfahren entspricht der Dickschicht-Hybrid-Technik. Das Elektrodenkonzept und eine entsprechend realisierte Elektrodenanordnung zeigt Bild 5.

Die beiden kammförmigen Elektroden 10, 11 entsprechen in ihrer Grundfunktionalität den beiden Kondensatorplatten, auf die mittlere Elektrode 12 wird die Elektronikmasse oder ein geeignetes Schirmsignal geschaltet.

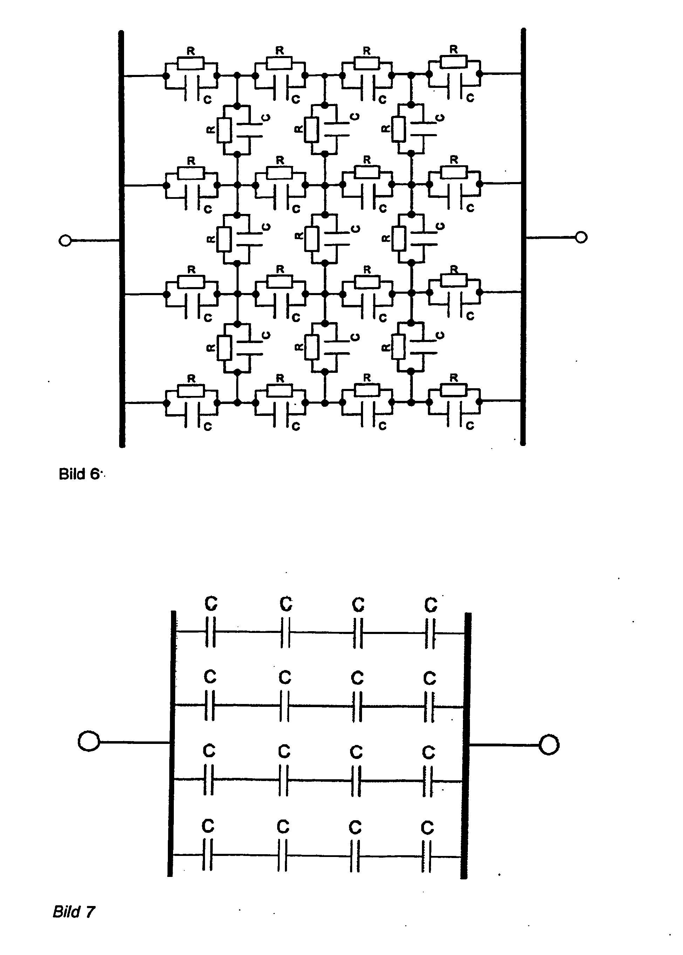

Bild 6 zeigt ein vereinfachtes elektrisches Ersatzschaltbild des Basissensors als ein Netzwerk aus einer R-C Kombination zwischen den Elektrodenplatten.

Unter Annahme eines homogenen Feldes liegen die Äquipotentiallinien innerhalb des Kondensators parallel zu den Elektroden. Über die senkrechten Zweige fließt daher kein Strom, sie können vernachlässigt werden.

Zur Vermeidung von Polarisationseffekten wird die Basissensoranordnung mit mittelwertfreien Wechselspannungssignalen beschaltet. Eine Messung mit sinusförmiger Wechselspannung ist dabei weniger geeignet, da der kapazitive Strom erheblich größer als die ohmsche Komponente des Stromes wäre und die Stromkomponenten nur mit erheblichem Elektronikaufwand getrennt werden können.

Für die Messung der Leitfähigkeit wird daher an die Elektroden eine abschnittsweise konstante Spannung angelegt. Im eingeschwungenen Zustand fließt über die Kondensatoren des Ersatzschaltbildes im Zeitabschnitt mit konstanter Eingangsspannung kein Strom. Während der Leitfähigkeitsmessung liegt eine konstante Spannung an den Elektroden an. Im Gleichspannungsersatzschaltbild können daher zudem die Kondensatoren gestrichen werden. Aus der Serien- und Parallelschaltung ergibt sich der resultierende Ersatzwiderstand R. Aus diesem wird dann mit Kenntnis der Geometrie der Elektrodenanordnung, bzw. der Zellkonstante $k_z$ die Leitfähigkeit $\kappa$ bestimmt.

Für den Fall einer konstanten elektrischen Feldstärke E zwischen den Elektroden ist der Sensorwiderstand:

$$R_x = \frac{\vec{E} \cdot \int_0^l d\vec{s}}{\kappa \cdot \vec{E} \cdot \int d\vec{A}} = \frac{l}{A \cdot \kappa}$$

und mit der Zellkonstanten

$$k_z = \frac{l}{A}$$

die elektrische Leitfähigkeit

$$\kappa = \frac{k_z}{R}$$

Der Quotient $\dfrac{l}{A}$ ist die Zellkonstante. Sie ist ausschließlich von der Geometrie der Sensoranordnung abhängig.

Abrieb, Ionen, zerschlagene Ölmoleküle, Säuren, Ölseifen usw. bewirken eine Erhöhung der Leitfähigkeit $\kappa$ der Öle. Die elektrische Leitfähigkeit wächst mit steigender Ionenkonzentration und Ionenbeweglichkeit. Die elektrische Leitfähigkeit nahezu aller Verschmutzungsprodukte ist groß im Vergleich zu der extrem geringen Leitfähigkeit reiner Öle. Es besteht ein direkter Zusammenhang zwischen der elektrischen Leitfähigkeit und dem Verschmutzungsgrad der Öle. Eine Vergrößerung der elektrischen Leitfähigkeit von Ölen im Betrieb kann als eine steigende Ölverschmutzung oder größerer Ölverschleiß interpretiert werden.

Durch Ölqualitätsveränderungen bzw. dabei erzeugte Ionen und Fremdpartikel werden einzelne Zweige im Ersatzschaltbild quasi kurzgeschlossen und die gemessene elektrische Leitfähigkeit und die gemessene Dielektrizitätszahl verändert.

Die Messung der Dielektrizitätszahl kann erfindungsgemäß über dieselben Basissensoranordnungen und Sensorelektroniken erfolgen, über die auch die elektrische Leitfähigkeit bestimmt wird.

Die Kapazität $C_x$ wird über die Definitionsgleichung der Kapazität

$$C = \frac{Q}{U} = \frac{\oint \vec{D} * d\vec{A}}{\int_0^l \vec{E} * d\vec{s}} = \frac{\oint \varepsilon * \vec{E} * d\vec{A}}{\int_0^l \vec{E} * d\vec{s}}$$

berechnet. Die Kapazität ist der Verschiebungsfluss zwischen zwei Elektroden dividiert durch die Spannung zwischen den Elektroden.

Bei räumlich konstanter elektrischer Feldstärke $E$ kann diese wiederum vor das Integral gezogen und gekürzt werden:

$$C = \frac{\varepsilon * \oint d\vec{A}}{\int_0^l ds} = \frac{\varepsilon \cdot A}{l}$$

bzw.

$$\varepsilon_r = \frac{C \cdot l}{A \cdot \varepsilon_0} = \frac{C}{k_z \cdot \varepsilon_0}$$

A

Der Quotient $\dfrac{A}{l}$ ist wiederum die Zellkonstante $k_z$. Die Kapazität ist (bei konstanter Dielektrizitätszahl $\varepsilon_r$) nur durch die geometrische Form des Kondensators und die dielektrischen Materialeigenschaften bestimmt.

Die Dielektrizitätszahl von Ölen liegt in einer Größenordnung von $\varepsilon_r \approx 2{,}2$, die Dielektrizitätszahl von Wasser ist etwa $\varepsilon_r \approx$ **81**. Der Wassergehalt in Öl kann über die Messung der Dielektrizitätszahl gemessen werden. Bei als Kühlschmierstoff eingesetzten Ölen soll, wegen des mit steigendem Wassergehalt größer werdenden Ölverschleißes, der Wassergehalt der Öle über eine genaue Messung der Dielektrizitätszahl zusätzlich gemessen werden. Zudem kann mit dieser Information der Einfluss des Wassers auf die Leitfähigkeitsänderung der Öle kompensiert werden. In anderen Anwendungen ermöglicht die gemessene Dielektrizitätszahl eine Aussage über den Abbau von Additiven im Öl.

Nach dem Anlegen einer höher frequenten Wechselspannung an die Elektroden ist der über die Kondensatoren des Ersatzschaltbildes fließende Strom wesentlich größer als der Strom über die hochohmigen Widerstände des Öls. Als vereinfachtes Wechselspannungsersatzschaltbild kann dann das in Bild 7 skizzierte Ersatzschaltbild angenommen werden.

Die Einzelkondensatoren können wiederum zu einer gemeinsamen Kapazität C zusammengefasst werden. Mit Kenntnis der Sensorgeometrie bzw. der Zellkonstante **$k_z$** wird daraus die gesuchte Dielektrizitätszahl bestimmt.

Der Basissensor ist eine Elektrodenanordnung, bei der das zu messende Öl bei der Leitfähigkeitsmessung als elektrischer Leiter und bei der Dielektrizitätszahlmessung als Dielektrikum dient. Öl ist ein elektrischer Nichtleiter. Die extreme Hochohmigkeit der Basissensoren und die daraus resultierenden extrem kleinen Messströme bedingen eine hohe Störempfindlichkeit gegenüber eingestreuten elektromagnetischen Feldern. Durch eine Integration der extrem kleinen Ströme wird eine ausreichende Störunterdrückung erreicht.

Der Widerstand und die Kapazität sollen unabhängig voneinander gemessen werden. Zur Vermeidung von Polarisationseffekten wird der Basissensor mit mittelwertfreien Testsignalen beaufschlagt. Anderenfalls würden zusätzliche Polarisationsspannungen das Messergebnis verfälschen oder sogar die Messung unmöglich machen. Die Verwendung sinusförmiger Testsignale ist wegen des extrem hochohmigen Widerstandes $R_x$ und den geringen ohmschen Stromkomponenten im Vergleich zu den kapazitiven Verschiebungsströmen über die Sensorkapazität $C_x$ problematisch. Es soll daher mit getakteten, mittelwertfreien, abschnittsweise konstanten Testsignalen die Kapazität und der Widerstand des Basissensors gemessen werden. Hierzu wird an die Elektroden eine abschnittsweise konstante Spannung angelegt und der verursachte elektrische Strom gemessen. Zur Erreichung einer hohen Störunterdrückung und damit letztlich auch einer hohen Genauigkeit wird hierzu der Strom über die Zeit integriert und zur Leitfähigkeitsmessung ausgewertet.

Der hochohmige Basissensor erfordert daher eine außerordentlich empfindliche und dennoch sehr störsichere Elektronik. Beides kann durch eine integrierende Messelektronik und eine umgehende Umwandlung der Messinformationen in digitale Signale erreicht werden. Hierzu werden der Basissensorwiderstand und die Basissensorkapazität über Operationsverstärkerschaltungen (OPs) in Frequenz- bzw. Zeitsignale umgewandelt. Die Zeitmessungen erfolgen über einen Mikrocontroller, der zudem die digitale Signalvorverarbeitung übernimmt.

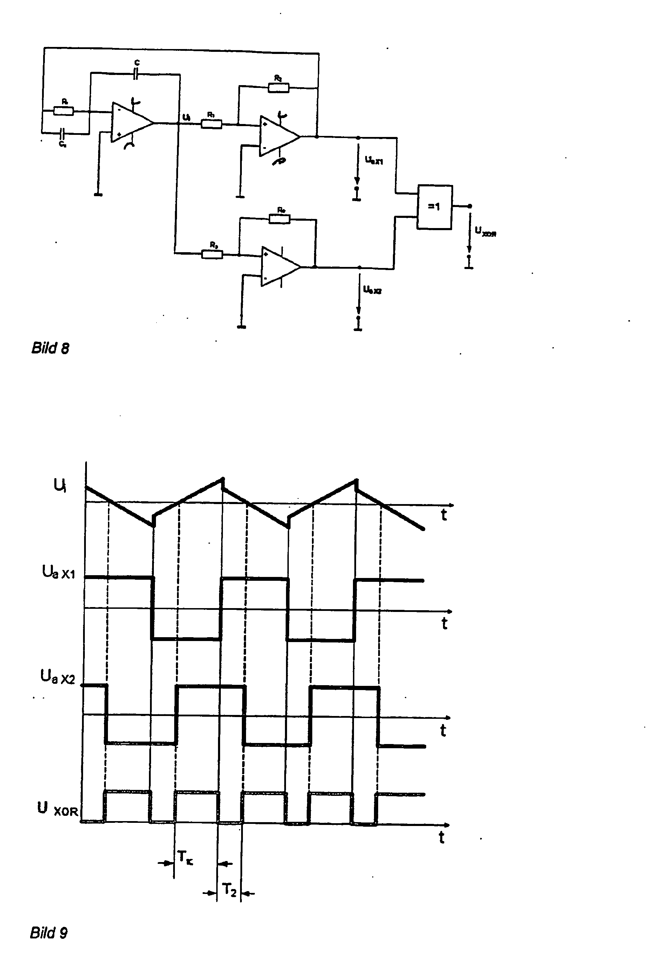

Die analoge Sensorelektronik (siehe Bild 8) besteht aus einem Relaxationsoszillator, wobei der Basissensor in den Eingangskreis des Integrators geschaltet ist. Aus dem analogen Ausgangssignal des Integrators werden über einen Mikrocontroller sowohl die elektrische Leitfähigkeit als auch die Dielektrizitätszahl bestimmt. Hierzu werden den gesuchten Messgrößen proportionale Zeitintervalle des Sensorsignals gemessen. Dabei muss eine, noch zu bestimmende, minimale Restleitfähigkeit $k_{min}$ überschritten werden.

Die Ausgangssignale des Integrators $u_i$ der beiden Schmitt-Trigger $u_{aX1}$ und $u_{aX2}$, der untere Schmitt-Trigger arbeitet als Komparator, und sind in Bild 9 skizziert.

Die Ausgangsspannungen der beiden Schmitt-Trigger werden auf ein XOR-Gatter geschaltet. Dessen Ausgangssignal beinhaltet die mit $T_k$ bezeichnete high-Zeit, sie ist dem spezifischen Widerstand $\rho$ proportional und umgekehrt proportional zur elektrischen Leitfähigkeit:

$$\kappa = \frac{R_1}{R_2} \cdot \frac{l}{A} \cdot \frac{C}{T_\kappa}$$

Die Differenz aus $T_k$ und $T_2$ ist proportional zur Dielektrizitätszahl:

$$\varepsilon_r = (T_\kappa - T_2) \cdot \frac{\kappa}{2 \cdot \varepsilon_0}$$

Nachfolgend werden diese beiden Gleichungen erläutert. Während der Zeit $T_k$ wird die elektrische Leitfähigkeit des Öls gemessen. Die Eingangsspannung des Integrators und damit auch die Spannung über der Sensorkapazität $C_x$ ist in diesem Zeitintervall konstant. Hieraus folgt für eine zeitlich quasi konstante Kapazität unmittelbar

$$i_{C_x} = C_x \cdot \frac{dU}{dt} = 0 \; .$$

Für die Integratorausgangsspannung $u_I$ folgt damit zum Umschaltpunkt des Schmitt-Triggers

$$u_I = -\frac{1}{R_x \cdot C} \int_0^{T_K} U_{ax_1}(\tau) d\tau$$

und für $U_{aX_1} = konst.$

$$u_I = -\frac{1}{R_x \cdot C} \cdot U_{ax_1} \cdot T_K \; .$$

Die Detektion des Nulldurchgangs der Integratorausgangsspannung erfolgt über den unten im Schaltbild dargestellten, als Schmitt-Trigger mit sehr kleiner Hysterese beschalteten Komparator. Der Umschaltpunkt des Schmitt-Triggers ist über

$$u_I = \frac{R_1}{R_2} \cdot U_{ax_1}$$

gegeben. Eingesetzt in die obige Gleichung errechnet sich

$$R_x = \frac{R_2}{R_1} \cdot \frac{T_\kappa}{C}$$

oder für die Leitfähigkeit:

$$K = \frac{R_1}{R_2} \cdot \frac{l}{A} \cdot \frac{C}{T_K}$$

Durch die parallele Kapazität $C_x$ des Sensors wird ein Sprung der Integratorausgangsspannung verursacht. Hieraus wird die Dielektrizitätszahl bestimmt.

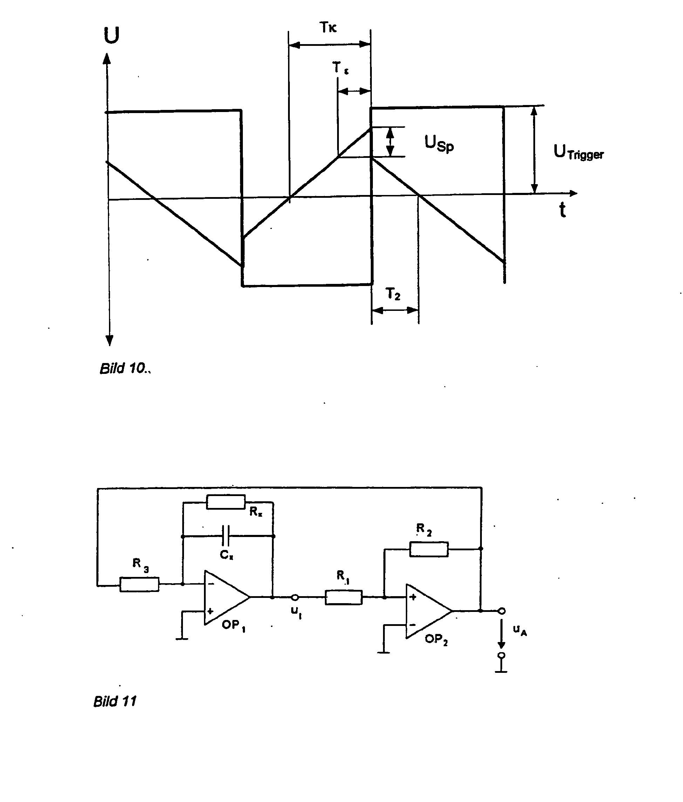

<u>Bild 10</u> zeigt den Zusammenhang zwischen $T_k$ und $T_\varepsilon$

Zur Berechnung benötigt man die Zeit $T_\varepsilon$. Dieses entspricht der Integrationszeit für die Integration über die durch $C_x$ verursachte Sprunghöhe $U_{Sp}$. Sie kann aus der Differenz der Zeiten $T_k$ und $T_2$ berechnet werden.

$$T_\varepsilon = T_k - T_2$$

Die Zeiten $T_k$ und $T_2$ werden in der Sensorelektronik durch das der analogen Sensorelektronik nachgeschaltete XOR-Gatter ermittelt (siehe Bild 10). $T_k$ ist dann die high-Zeit und $T_2$ die Low-Zeit der Ausgangsspannung des XOR-Gatters.

Zum Zeitpunkt der Umschaltung der Ausgangsspannung des Schmitt-Triggers und damit auch der Eingangsspannung des Integrators werden die beiden Kondensatoren $C_x$ und $C$ umgeladen. Sie bilden einen kapazitiven Spannungsteiler, der über die Gleichung

$$\frac{2 \cdot U_{Trigger}}{C} = \frac{U_{Sprung}}{C_x}$$

beschrieben werden kann.

Aus der Gleichung für die Ausgangsspannung des Integrators folgt

$$U_{Sprung} = \frac{T_\varepsilon}{R_x \cdot C} \cdot U_{Trigger}$$

und

$$\frac{2 \cdot U_{Trigger}}{C} \cdot C_x = \frac{T_\varepsilon}{R_x \cdot C} \cdot U_{Trigger}$$

folgt

$$C_x = \frac{T_\varepsilon}{2 \cdot R_x}$$

bzw.

$$\varepsilon_r = (T_\kappa - T_2) \cdot \frac{\kappa}{2 \cdot \varepsilon_0}$$

Hierüber wird die Dielektrizitätszahl $\varepsilon_r$ aus der Leitfähigkeit $\kappa$ und der Zeit $T_\varepsilon$ berechnet.

Mit Hilfe der Sensorelektronik wird aus dem einem Sensorsignal sowohl die elektrische Leitfähigkeit als auch die Dielektrizitätszahl der Öle bestimmt. Allerdings ist eine Mindestleitfähigkeit auch für die Messung der Dielektrizitätszahl erforderlich. In einer speziellen Ausgestaltung der Elektronik wird durch den Basissensoranschluss parallel geschaltete, sehr hochohmige Widerstände eine Mindestleitfähigkeit simuliert. Von den gemessenen Leitfähigkeiten wird dann die simulierte Leitfähigkeit wieder subtrahiert.

Zur Kompensation von Herstellungstoleranzen des Basissensors kann dessen Kalibrierung durch eine Messung der Kapazität des mit hochtrockener Luft oder hochreinem Stickstoff gefüllten Basissensors erfolgen. Auswirkungen mechanischer Fertigungstoleranzen auf das Messergebnis können so eliminiert werden. Da bei der oben beschriebenen Elektronikschaltung eine Mindestleitfähigkeit vorausgesetzt werden muss, wird hierfür entweder eine Mindestleitfähigkeit über die parallel zum Basissensor geschalteten Hochohmwiderstände simuliert, oder eine modifizierte Elektronikschaltung genutzt, bei der die Integrationskapazität C durch den Basissensor ersetzt, also in den Rückkoppelzweig des Integrators geschaltet wird.

Bild 11 verdeutlicht die Schaltung mit einem Relaxationsoszillator.

Während bei der ersten Schaltung (Bild 8) die Elektrizitätszahl $\varepsilon_r$ bzw. die Sensorkapazität $C_x$ während des Umschaltsprungs, also bei sehr hohen Frequenzen gemessen wird, dient die Sensorkapazität $C_x$ bei der in Bild 11 dargestellten analogen Sensorelektronikschaltung zur Integration eines abschnittsweise konstanten Stromes. Hier wird die Sensorkapazität $C_x$ bei niedrigen Frequenzen gemessen. Der parallele Sensorwiderstand kann hier vernachlässigt werden, da der Strom durch den Kondensator sehr viel größer ist als der Strom durch $C_x$. Eine mögliche Anwendung, bei der die Dielektrizitätszahlmessung bei verschiedenen Frequenzen eingesetzt werden kann, sind Kraftstoffsensoren für Multi-Fuel-Fahrzeuge oder die Charakterisierung verschiedener Öle und/oder Ölkomponenten. Andere Anwendungsbereiche sind direkte Gasfeuchtemessanordnungen auch aggressiver Gase oder Gasfeuchtemessungen mit sehr einfachen Elektroden, z.B. auf der Innenseite von Fahrzeugscheiben und gegebenenfalls gleichzeitige Luftfeuchte und Betauungsmessungen.

Die Ausgangsspannung des Integrators ist bei konstanter Eingangsspannung $u_A$ und $u_I(0) = 0$:

$$u_I(t) = -\frac{u_A}{R_2 \cdot C} \cdot t$$

Der mit OP$_2$ aufgebaute Schmitt-Trigger liefert die vom Umkehrintegrator aufintegrierte, abschnittsweise konstante Ausgangsspannung $u_{Amin}$ bzw. $u_{Aamin}$. Erreicht die Integratorspannung $u_I$ den Trigger-Pegel des Schmitt-Triggers $|U_{IS}|$, ändert die zu integrierende Spannung $u_a$ ihr Vorzeichen. Dadurch läuft der Ausgang des Integrators in umgekehrter Richtung, bis der andere Trigger-Pegel erreicht ist.

Die Umschaltung des Schmitt-Triggers erfolgt bei den Schaltspannungen

$$|U_{IS}| = \left| -\frac{R_1}{R_2} \cdot U_{a,min} \right| = \frac{R_1}{R_2} \cdot U_{a,max} = \frac{R_1}{R_2} \cdot \left| U_{a,max/min} \right|$$

Es folgt für

$$t = \frac{T}{4}$$

$$|U_{IS}| = \frac{|U_{a,max/min}|}{R_3 \cdot C} \cdot \frac{T}{4}$$

T ist die Periodendauer der Dreieck- und der Rechteckschwingung und direkt proportional zur gesuchten Kapazität des Basissensors und zur Dielektrizitätszahl:

$$T = \frac{1}{f} = \frac{4R_1 R_3}{R_2} \cdot C$$

$$\varepsilon = T \cdot \frac{R_2}{4R_1 R_3} \cdot \frac{l}{A}$$

Die direkte Messung der Dielektrizitätszahl des Fluides zwischen den beiden Elektroden ermöglicht in Verbindung mit der hochauflösenden Sensorelektronik sowohl eine Feuchtebestimmung als auch die genaue Kalibrierung der Basissensoren.

Die Eingangsströme der Operationsverstärker bewirken in den analogen Sensorelektronikschaltungen eine Verschiebung des Tastverhältnisses der Ausgangssignale $u_a$. Die Frequenz wird dagegen nicht verändert. Da erfindungsgemäß jeweils über eine Periode oder ein ganzzahliges Vielfaches einer Periode von $u_a$ gemessen wird, wirken sich die Eingangsruhe- und Offsetströme nicht auf das Messergebnis aus.

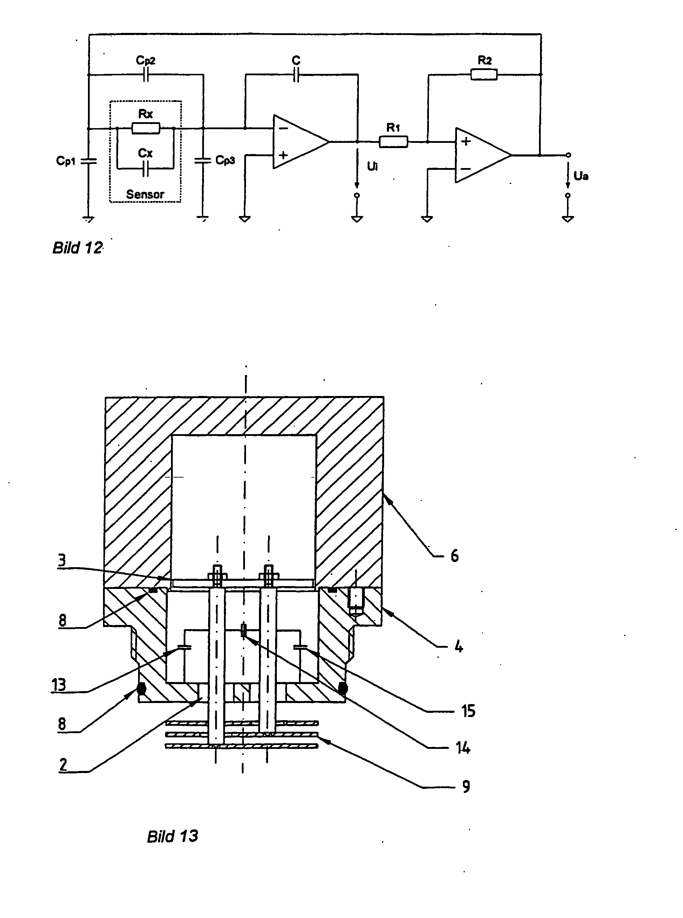

In Bild 12 sind die parasitären Kapazitäten der analogen Sensorelektronik eingetragen. Bild 13 zeigt die gleichen parasitären Kapazitäten in der Basissensoranordnung.

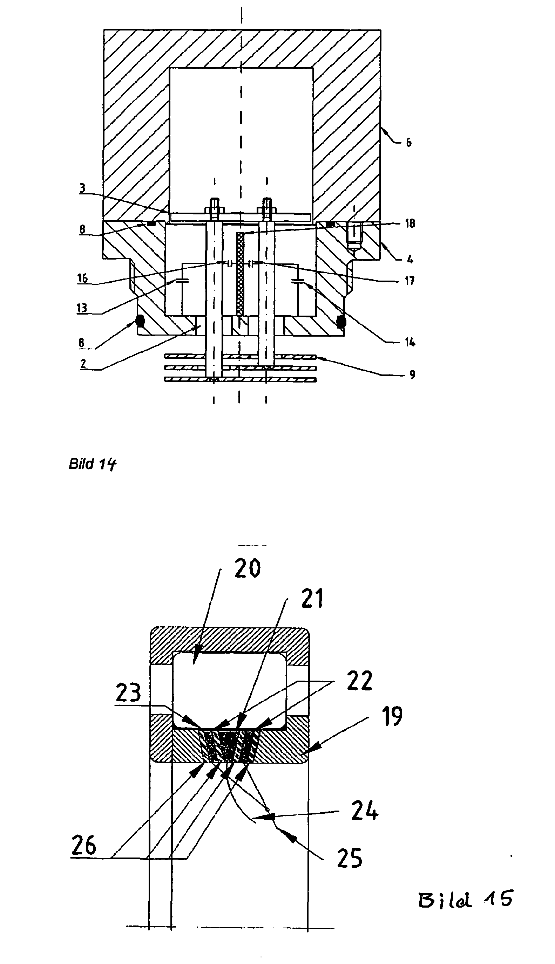

Die Kapazität $C_{P1}$ liegt parallel zum Ausgang des Schmitt-Triggers und hat keinen negativen Einfluss auf die Schaltung. $C_{P2}$ liegt parallel zur Sensorkapazität $C_X$. Zur Reduktion von $C_{P2}$ wird erfindungsgemäß ein Schirm eingefügt, die Kapazität und $C_{P2}$ wird dadurch den Kapazitäten $C_{P1}$ und $C_{P3}$ zugeordnet. $C_{P3}$ liegt wiederum zwischen der virtuellen Masse am invertierten Eingang des Operationsverstärkers und Masse. Es fällt also keine Spannung über $C_{P3}$ ab, $C_{P3}$ ist daher vernachlässigbar. Bild 14 zeigt die praktische Realisierung im Basissensor. Auf der Elektronikplatine wird erfindungsgemäß dasselbe Schirmkonzept über das Einfügen von Masseleiterbahnen umgesetzt.

Zur Vermeidung von Polarisationseffekten sind mittelwertfreie Testsignale erforderlich mit denen der Basissensor beaufschlagt wird. Erfindungsgemäß werden Rail-To-Rail Operationsverstärker mit symmetrischer Versorgungsspannung verwendet. Eine verbesserte Symmetrierung wird über einen Spannungsteiler mit zwei gleichen Widerständen zwischen den Versorgungsspannungen erreicht. Dessen Mittelabgriff dient als Bezugspotential der Sensorelektronik.

Durch die Bewegung des hier als elektrischer Leiter wirkenden Öls werden in das Öl, durch unvermeidbare Magnetfelder, nach dem Induktionsgesetz strömungsgeschwindigkeitsproportionale Störspannungen in die Elektrodenanordnung induziert. Diese sind dem eigentlichen Messsignal überlagert. Bereits das konstante Erdmagnetfeld kann diesen Effekt bewirken. Sich ändernde Magnetfelder werden sowohl bei den Durchflusssensoren als auch bei den Tauchsensoren weitere transformatorische Störspannungen induzieren. Die erfindungsgemäße Sensorelektronik kompensiert diese Störeffekte.

Die Ionenbeweglichkeit und damit auch die elektrische Leitfähigkeit sind von der inneren Reibung der Öle und damit auch von deren Temperatur abhängig. Die Leitfähigkeit steigt mit der Öltemperatur. Zur Bewertung der Ölverschmutzungen sind daher eine Temperaturkompensation der Leitfähigkeitsmesswerte und eine Umrechnung auf eine Referenztemperatur erforderlich. Zur Temperaturkompensation der Leitfähigkeitswerte wird zusätzlich die Temperatur vorzugsweise über einen PT100 gemessen. Dieser ist vorzugsweise an der Elektrodenhalterung auf der Elektronikseite der Glas-Metalldurchführung angeordnet. Die Umrechnung der gemessenen elektrischen Leitfähigkeit und der Dielektrizitätszahl erfolgt erfindungsgemäß vorzugsweise über eine adaptive, selbstlernende Temperaturkompensation.

Die Berechnung der elektrischen Leitfähigkeit und der Dielektrizitätszahl bei der Referenztemperatur $T_0$ kann über ein die Temperaturabhängigkeit approximierendes Polynom der Form

$$\kappa_{T0} = \kappa_{T0a} + \left(a\Delta T_i + b\Delta T_i^{\,2} + c\Delta T_i^{\,3}\right)\cdot \kappa_m$$

erfolgen. $K_m$ ist der aktuelle Messwert der elektrischen Leitfähigkeit bei der Temperatur $T_i$. $\kappa$ **T0** ist die angenäherte elektrische Leitfähigkeit des Öles bei der Referenztemperatur $T_0$, $\kappa_{T0a}$ ist die vorher berechnete (alte) elektrische Leitfähigkeit bei der Referenztemperatur $T_0$, a, b und c sind die adaptiv zu bestimmenden Koeffizienten des approximierenden Polynoms,

$$\Delta T_i = T_0 - T_i$$

ist die Temperaturdifferenz. Die Annäherung durch ein Polynom dritten Grades gewährleistet eine gute Annäherung bei vertretbar geringem Rechenaufwand für den eingesetzten Mikrorechner. Für die Bestimmung der Koeffizienten des Polynoms wird auf der Basis der Gaußschen Methode der kleinsten Fehlerquadrate aus den N Messwertepaaren und dem approximierenden Polynom eine Risikofunktion definiert, deren Minimierung eine Bestimmung der gesuchten Koeffizienten ermöglicht.

Es ergeben drei linear unabhängige Gleichungen für die drei gesuchten Koeffizienten

$$\begin{pmatrix} \kappa_m \sum_{i=1}^{N}\Delta T_i^2 & \kappa_m \sum_{i=1}^{N}\Delta T_i^3 & \kappa_m \sum_{i=1}^{N}\Delta T_i^4 \\ \kappa_m \sum_{i=1}^{N}\Delta T_i^3 & \kappa_m \sum_{i=1}^{N}\Delta T_i^4 & \kappa_m \sum_{i=1}^{N}\Delta T_i^5 \\ \kappa_m \sum_{i=1}^{N}\Delta T_i^4 & \kappa_m \sum_{i=1}^{N}\Delta T_i^5 & \kappa_m \sum_{i=1}^{N}\Delta T_i^6 \end{pmatrix}\begin{pmatrix} a \\ b \\ c \end{pmatrix} = \begin{pmatrix} \sum_{i=1}^{n}(\kappa_{T0} - \kappa_{T0a})\Delta T_i \\ \sum_{i=1}^{n}(\kappa_{T0} - \kappa_{T0a})\Delta T_i^{\,2} \\ \sum_{i=1}^{n}(\kappa_{T0} - \kappa_{T0a})\Delta T_i^{\,2} \end{pmatrix}$$

Hieraus werden die drei gesuchten Koeffizienten z.B. mit Hilfe der Cramerschen Regel bestimmt.

In einer weiteren Ausgestaltung der Erfindung wird ein, ggf. zusätzlicher, erfindungsgemäßer Basissensor so realisiert, dass das Volumen zwischen den Elektroden der Schmierfilm im zu überwachenden Lager ist. In Bild 15 ist eine solche Anordnung skizziert.

Über die gemessene Kapazität C wird hier, gegebenenfalls zusätzlich, die Schmierspaltdicke 1 bestimmt.

$$l = \frac{\varepsilon \cdot A}{C}$$

Eine wichtige Ergänzung des Ölsensorsystems ist die Einbindung in eine geeignete Kommunikationsstruktur und die Realisierung eines Monitoringsystems. Einsatzbereiche des Sensorsystems sind dezentral angeordnete energie-, fertigungs- und automatisierungstechnische Anlagen wie z.B. Windturbinen, Transformatorstationen,

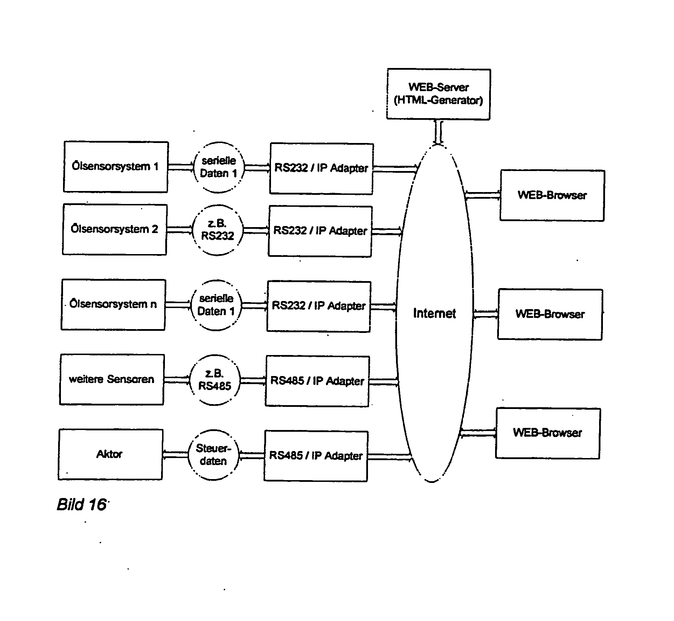

Hydraulikanlagen, Getriebe usw. Hierfür wird eine Online-Überwachung dezentraler Anlagen über ein vorzugsweise Web-basiertes Monitoringsystem realisiert. Das nachfolgende Bild 16 zeigt das Instanzennetz eines solchen Monitoringsystems.

Das Web-basierte, dezentrale Monitoringsystem, bei dem die Sensordaten vorzugsweise über das Internet übertragen und über eine hinterlegte Datenbank in eine als Bedienoberfläche des Monitoringsystems realisierte HTML-Seite eingetragen werden, ermöglicht die Überwachung der Anlagen sowohl durch die Anlagenbetreiber, als auch durch mit der Wartung beauftragte Dienstleister.

In einer weiteren Ausgestaltung der Erfindung wird in Anlehnung an die Patentschrift DE 19914658 eine Messung des Impedanzspektrums des Basissensors mit mittelwertfreien pseudozufälligen Binär- oder Ternärsignalen vorgeschlagen. Anstelle des abschnittsweise konstanten Ausgangssignals des Schmitt-Triggers des Relaxationsoszillators werden hier abschnittsweise konstante digitale Rauschsignale auf den Basissensor im Eingangszweig des Integrators geschaltet. Die Kreuzkorrelationsfunktion $\theta_{xy}(\tau)$ aus dem Eingangssignal x(t) und Ausgangssignal y(t) eines linearen, zeitinvarianten Übertragungsgliedes, hier des Basissensors, ist gleich dem Faltungsprodukt aus der Impulsantwort des

Übertragungsgliedes $g(\tau)$ und der Autokorrelationsfunktion $\varphi_{xx}(\tau)$

$$\varphi_{xy}(\tau) = g(\tau) * \varphi_{xx}(\tau)$$

oder in ausführlicher Schreibweise:

$$\varphi_{xy}(\tau) = \int_{-\infty}^{+\infty} g(t) \cdot \varphi_{xx}(t - \tau)dt$$

Dies ergibt sich aus dem Einsetzen des Dahamelintegrals in die aus dem Eingangs- und Ausgangssignal gebildete Kreuzkorrelationsfunktion und Vertauschen der Integrationsreihenfolge. Ist das Eingangssignal ein periodisches Pseudozufallssignal, so kann die Integration über eine Periode erfolgen. Die Impulsantwort eines Kondensators mit dem Kondensatorstrom i als Eingangssignal und der Kondensatorspannung u als Ausgangssignal ist

$$g(t) = \frac{1}{C}$$

Die gesuchte Kapazität errechnet sich also über

$$C = \frac{\int_{-0}^{+T} \varphi_{ii}(\tau)d\tau}{\varphi_{iu}(\tau)}$$

Das Integral über die Autokorrelationsfunktion des pseudozufälligen Kondensatorstromes ist bekannt. Die Berechnung der Kreuzkorrelationsfunktion $\varphi_{iu}(\tau)$ zwischen dem pseudozufälligen periodischen Kondensatorstrom i(t) und der Kondensatorspannung u(t) wird bei jeder Messung numerisch mit dem Mikrorechner durchgeführt. Wegen des digitalen Eingangssignals i(t) wird die Multiplikation in Verbindung mit der Integration eine einfache Summation. Bei der Wahl schnell veränderlicher Eingangssignale kann das Ausgangssignal auch eine Periode oder eine ganz zahlige Anzahl von Perioden später gemessen werden.

Anstelle der Kapazität kann auch die Impulsantwort g(t) oder nach der Anwendung einer schnellen Fouriertransformation (FFT) die Übertragungsfunktion des Basissensors bestimmt werden.

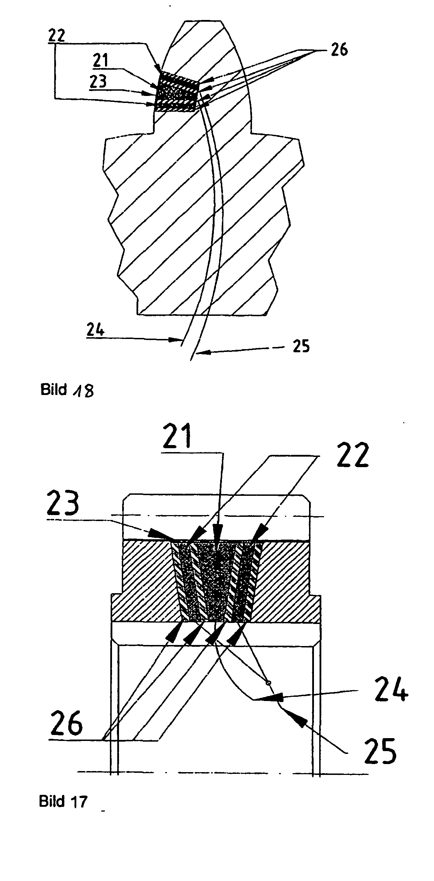

In Bild 17 und 18 ist eine Realisierung ähnlich Bild 15 gezeigt, allerdings bei Anordnung der Elektroden 21,22 an der Zahnflanke eines Zahnes eines Zahnrades, wobei der Ölspalt 23 zum kämmenden Gegenelement sowie die Zuleitungen 24,25 und die Isolierung 26 verdeutlicht ist.

Legende

1  2-Platten-Basissensor
2  Glas-/Metall-Durchführung
3  Elektronikplatine
4  Sensorträger
5  Sensortopf
6  Sensordeckel
7  Ein-/Auslassöffnung
8  O-Ring
9  3-Platten-Basissensor
10  Kammelektrode 1

| 11 | Kammelektrode 2 |
| 12 | Schirmelektrode |
| 13 | parasitäre Kapazität $Cp_1$ |
| 14 | parasitäre Kapazität $Cp_2$ |
| 15 | parasitäre Kapazität $Cp_3$ |
| 16 | parasitäre Kapazität $Cp_{21}$ |
| 17 | parasitäre Kapazität $Cp_{22}$ |
| 18 | Schirm |
| 19 | Lager-Innenring |
| 20 | Wälzlagerkörper |
| 21 | Elektrode 1 |
| 22 | Elektrode 2 |
| 23 | Ölspalt |
| 24 | Zuleitung Elektrode 1 |
| 25 | Zuleitung Elektrode 2 |
| 26 | Isolierung |

## Patentansprüche

1. Verfahren zur Messung physikalischer Größen von elektrisch gering leitenden und nicht leitenden Fluiden, wobei sich das Fluid zwischen elektrisch leitenden Elektroden eines Basissensors (1,9) befindet, wobei auf den Basissensor (1,9) ein Testsignal in Form einer getakteten Gleichspannung oder eines getakteten Gleichstroms geschaltet wird, der sich einstellende Ausgangsstrom oder die Ausgangsspannung mittels eines Integrators aufintegriert wird, dessen Ausgangssignal, gegebenenfalls über einen Verstärker, auf einen ersten Schmitt-Trigger geschaltet wird, dessen Ausgangssignal wiederum als Testsignal auf den Basissensor (1,9) geschaltet wird,
**dadurch gekennzeichnet, dass** das Ausgangssignal zudem auf einen zweiten Schmitt-Trigger geschaltet wird, der parallel zu dem ersten Schmitt-Trigger geschaltet ist, und der als Komparator dient, wobei das Ausgangssignal beider Schmitt-Trigger auf ein XOR-Gatter geschaltet ist, aus dessen Ausgangssignalen über einen Mikrorechner die zu messenden physikalischen Größen des Fluids errechnet werden und einer Auswertelogik zugeführt werden, wobei aus dem gemessenen Signal sowohl der Widerstand als auch die Kapazität bestimmt wird.

2. Verfahren nach Anspruch 1,
**dadurch gekennzeichnet, dass** das Testsignal in Form von getakteten, mittelwertfreien Messsignalen aufgeschaltet wird.

3. Verfahren nach Anspruch 1 oder 2,
**dadurch gekennzeichnet, dass** auf eine erste Sensorelektrode des Basissensors (1,9) das Testsignal in Form einer abschnittsweise konstanten Spannung oder eines abschnittsweise konstanten Stroms geschaltet wird und eine zweite Sensorelektrode des Basissensors (1,9) auf den invertierenden Eingang eines als Operationsverstärker ausgebildeten Integrators geschaltet wird, oder alternativ auf einen spannungsgegengekoppelten Operationsverstärker geschaltet wird.

4. Schaltungsanordnung zur Durchführung des Verfahrens gemäß einem der Ansprüche 1 bis 3, bestehend aus einem Basissensor (1,9), auf den eingangsseitig das Testsignal geschaltet ist und der ausgangsseitig an den invertierenden Eingang eines Operationsverstärkers als Integrator, gegebenenfalls über einen Verstärker, geschaltet ist, wobei das Ausgangssignal des Integrators an zwei parallel geschaltete Operationsverstärker als Schmitt-Trigger geschaltet ist, wobei das Ausgangssignal des ersten Schmitt-Triggers als Testsignal auf den Basissensor (1,9) geschaltet ist und auf ein XOR-Gatter geschaltet ist, auf das auch der Ausgang des zweiten Schmitt-Triggers geschaltet ist, wobei der Ausgang des XOR-Gatters an eine Auswertelogik geschaltet ist.

5. Verwendung des Verfahrens und/oder der Schaltungsanordnung nach einem der Ansprüche 1 bis 4,
**dadurch gekennzeichnet, dass** die gemessene elektrische Leitfähigkeit und/oder die Dielektrizitätszahl und die Änderung der elektrischen Leitfähigkeit und/oder die Änderung der Dielektrizitätszahl als Maß für eine Bewertung der Veränderung des Systems Öl-Maschine, Öl-Getriebe, Öl-Motor, Öl-Trafo usw. genutzt wird.

6. Verwendung nach dem Oberbegriff des Anspruches 5,
**dadurch gekennzeichnet, dass** über die gemessene elektrische Leitfähigkeit und/oder die Dielektrizitätszahl und/oder die Änderung der elektrischen Leitfähigkeit und/oder der Dielektrizitätszahl der Maschinenverschleiß, Getriebe-, Motor-, Transformatorenverschleiß usw. und der Ölverschleiß gemessen wird, oder unmittelbar oder mittelbar der Lager- oder Zahnflankenverschleiß gemessen wird.

**7.** Verwendung nach dem Oberbegriff des Anspruches 5,
**dadurch gekennzeichnet, dass** über die gemessene elektrische Leitfähigkeit und/oder die Änderung der elektrischen Leitfähigkeit und oder die gemessene Dielektrizitätszahl und/oder Änderung der Dielektrizitätszahl vorbeugende Wartungsmaßnahmen bereits lange vor vorbeugenden Instandsetzungen indiziert werden.

**8.** Verwendung nach dem Oberbegriff des Anspruches 5,
**dadurch gekennzeichnet, dass** über die Dielektrizitätszahl und/oder die Änderung der Dielektrizitätszahl der Wassergehalt der Öle und/oder mit der Änderung der Dielektrizitätszahl und/oder der Änderung der elektrischen Leitfähigkeit der Abbau von Additiven gemessen wird.

**9.** Verwendung nach dem Oberbegriff des Anspruches 5,
**dadurch gekennzeichnet, dass** das Volumen zwischen den Elektrodenplatten des Basissensors der Schmierfilm in einem zu überwachenden Lager ist oder der Schmierfilm zwischen Zahnflanken von Zahnrädern.

**10.** Verwendung nach Anspruch 9,
**dadurch gekennzeichnet, dass** zusätzlich oder alternativ die Schmierfilmdicke gemessen wird.

**11.** Verfahren nach einem der Ansprüche 1 bis 3,
**gekennzeichnet durch** eine adaptive selbstlernende Temperaturkompensation.

**12.** Verfahren nach einem der Ansprüche 1 bis 3,
**dadurch gekennzeichnet, dass** die Messsignale über eine serielle Schnittstelle, ein Bussystem und/oder eine LAN oder WLAN-Schnittstelle an ein vorzugsweise Web-basiertes Monitoringsystem geschickt werden.

**13.** Verfahren nach einem der Ansprüche 1 bis 3,
**gekennzeichnet durch** ein Web-basiertes Monitoringsystem.

**14.** Verwendung nach dem Oberbegriff des Anspruches 5,
**dadurch gekennzeichnet, dass** das Sensorsystem zur direkten Feuchtemessung auch von aggressiven und/oder korrosiven Gasen genutzt wird.

**15.** Verwendung nach dem Oberbegriff des Anspruches 5,
**gekennzeichnet durch** einen Einsatz als Kraftstoffsensor für Multifuel-Fahrzeuge und/oder - Maschinen.

**16.** Verwendung nach dem Oberbegriff des Anspruches 5,
**gekennzeichnet durch** einen Einsatz als Erdölsensor zur Messung von Parametern von Erdölen und deren Beiprodukte.

**17.** Verwendung nach dem Oberbegriff des Anspruches 5,
**gekennzeichnet durch** die Messung von Erdölinhaltsstoffen wie mechanische Verschmutzungen, Salzen, Säuren, Wasser, sich bildende Ölseifen usw. über Messungen der elektrischen Leitfähigkeit und der Dielektrizitätszahl des Erdöls.

**Claims**

**1.** A method for measuring physical parameters in electrically poorly conductive and non-conductive fluids, the fluid being located between electrically conductive electrodes of a base sensor (1, 9), a test signal in the form of a clocked d.c. voltage or a clocked d.c. current being supplied to the base sensor (1, 9), the resultant output current or output voltage being integrated by an integrator, the output signal of which is supplied, if applicable via an amplifier, to a first Schmitt trigger, the output signal of which is in turn supplied as a test signal to the base sensor (1, 9), **characterized by** that the output signal is in addition supplied to a second Schmitt trigger, which is connected in parallel to the first Schmitt trigger and which serves as a comparator, the output signal of the two Schmitt triggers being supplied to an XOR gate, from the output signals of which, via a microcomputer, the physical parameters to be measured in the fluid are calculated and fed to an evaluation logic system, wherein the resistance as well as the capacitance are determined from the measured signal.

2. The method according to claim 1, **characterized by** that the test signal is supplied in the form of clocked zero-mean measuring signals.

3. The method according to claim 1 or 2, **characterized by** that the test signal is supplied in the form of a sectionally constant voltage or a sectionally constant current to a first sensor electrode of the base sensor (1, 9), and a second sensor electrode of the base sensor (1, 9) is connected to the inverting input of an integrator adapted as an operational amplifier, or alternatively is connected to a voltage-feedback operational amplifier.

4. A circuit assembly for carrying out the method according to one of claims 1 to 3, comprising a base sensor (1, 9), to the input side of which the test signal is supplied, and the output of which is connected to the inverting input of an operational amplifier acting as an integrator, if applicable via an amplifier, the output signal of the integrator being supplied to two parallely connected operational amplifiers as a Schmitt trigger, wherein the output signal of the first Schmitt trigger is supplied as a test signal to the base sensor (1, 9), and is supplied to an XOR gate, to which the output of the second Schmitt trigger is also connected, the output of the XOR gate being connected to an evaluation logic system.

5. An application of the method and/or of the circuit assembly according to one of claims 1 to 4, **characterized by** that the measured electrical conductivity and/or the dielectric constant, and the change in the electrical conductivity and/or the change in the dielectric constant is used as a measure for the evaluation of the change in the system oil/machine, oil/gear, oil/engine, oil/transformer, etc.

6. The application according to the preamble of claim 5, **characterized by** that by means of the measured electrical conductivity and/or the dielectric constant and/or the change in the electrical conductivity and/or the dielectric constant, the machine wear, gear, engine, transformer wear, etc., and the oil wear are measured, or the bearing or tooth flank wear is directly or indirectly measured.

7. The application according to the preamble of claim 5, **characterized by** that by means of the measured electrical conductivity and/or the change in the electrical conductivity and/or the measured dielectric constant and/or the change in the dielectric constant, preventive maintenance measures are indicated long before preventive repairs.

8. The application according to the preamble of claim 5, **characterized by** that by means of the dielectric constant and/or the change in the dielectric constant, the water content of the oils, and/or by means of the change in the dielectric constant and/or the change in the electrical conductivity, the degradation of additives is measured.

9. The application according to the preamble of claim 5, **characterized by** that the volume between the electrode plates of the base sensor is the lubricating film in a bearing to be monitored or the lubricating film between tooth flanks of gear wheels.

10. The application according to claim 9, **characterized by** that additionally or alternatively, the thickness of the lubricant film is measured.

11. The method according to one of claims 1 to 3, **characterized by** an adaptive self-learning temperature compensation.

12. The method according to one of claims 1 to 3, **characterized by** that the measuring signals are sent via a serial interface, a bus system and/or a LAN or WLAN interface preferably to a web-based monitoring system.

13. The method according to one of claims 1 to 3, **characterized by** a web-based monitoring system.

14. The application according to the preamble of claim 5, **characterized by** that the sensor system for a direct moisture measurement is also used for aggressive and/or corrosive gases.

15. The application according to the preamble of claim 5, **characterized by** using the sensor as a fuel sensor for multi-fuel vehicles and/or machines.

16. The application according to the preamble of claim 5, **characterized by** using the sensor as a crude oil sensor for measuring parameters of crude oils and of by-products thereof.

17. The application according to the preamble of claim 5, **characterized by** the measurement of crude oil ingredients, such as mechanical dirt, salts, acids, water, forming oil soaps, etc. by means of measurements of the electrical conductivity and of the dielectric constant of the crude oil.

**Revendications**

1. Procédé destiné à la mesure de grandeurs physiques dans des fluides peu conducteurs et non conducteurs, le fluide se trouvant entre deux électrodes conductrices de l'électricité d'un capteur de base (1, 9), un signal de test, sous la forme d'une tension continue cadencée ou d'un courant continu cadencé, étant appliqué au capteur de base (1, 9), le courant de sortie ou la tension de sortie qui s'établit étant intégré ou intégrée au moyen d'un intégrateur, dont le signal de sortie est appliqué, le cas échéant par l'intermédiaire d'un amplificateur, à un premier déclencheur de Schmitt, dont le signal de sortie est appliqué, en tant que signal de test, au capteur de base (1, 9), **caractérisé en ce que** le signal de sortie est en addition appliqué à un deuxième déclencheur de Schmitt, qui est raccordé en parallèle au premier déclencheur de Schmitt et qui sert de comparateur, le signal de sortie des deux déclencheurs de Schmitt étant appliqué à une porte XOR, à partir des signaux de sortie de celle-ci, par l'intermédiaire d'un micro-ordinateur, les grandeurs physiques à mesurer dans le fluide étant calculées et transmises à un logique d'évaluation, dans laquelle la résistance aussi bien que la capacitance sont déterminées à partir du signal mesuré.

2. Procédé selon la revendication 1, **caractérisé en ce que** le signal de test est appliqué sous la forme de signaux de mesure cadencés de moyenne zéro.

3. Procédé selon la revendication 1 ou 2, **caractérisé en ce que** le signal de test est appliqué à une première électrode du capteur de base (1, 9) sous la forme d'une tension constante sur des certains segments ou d'un courant constant sur des certains segments, et une deuxième électrode du capteur de base (1, 9) est raccordée à l'entrée inverseuse d'un intégrateur configuré comme amplificateur opérationnel, ou alternativement est raccordée à un amplificateur opérationnel à boucle fermée de tension.

4. Circuiterie destinée à exécuter le procédé selon une des revendications 1 à 3, comprenant un capteur de base (1, 9), au côté d'entrée duquel le signal de test est appliqué, et la sortie duquel est raccordée à l'entrée inverseuse d'un amplificateur opérationnel agissant comme intégrateur, le cas échéant par l'intermédiaire d'un amplificateur, le signal de sortie de l'intégrateur étant appliqué à deux amplificateurs opérationnels raccordés en parallèle et agissant comme déclencheur de Schmitt, le signal de sortie du premier déclencheur de Schmitt étant appliqué en tant que signal de test au capteur de base (1, 9), et étant appliqué à une porte XOR, à laquelle la sortie du deuxième déclencheur de Schmitt est aussi raccordée, la sortie de la porte XOR étant raccordée à une logique d'évaluation.

5. Utilisation du procédé et/ou de la circuiterie selon une des revendications 1 à 4, **caractérisée en ce que** la conductivité électrique mesurée et/ou la permittivité diélectrique, et la variation de la conductivité électrique et/ou la variation de la permittivité diélectrique est utilisée comme mesure pour l'évaluation de la variation du système huile/machine, huile/transmission, huile/moteur, huile/transformateur, etc.

6. Utilisation selon le préambule de la revendication 5, **caractérisée en ce qu'**au moyen de la conductivité électrique mesurée et/ou la permittivité diélectrique et/ou la variation de la conductivité électrique et/ou de la permittivité diélectrique, l'usure de la machine, de la transmission, du moteur, du transformateur, etc., et l'usure de l'huile est mesurée, ou l'usure des roulements ou des flancs de dents est mesurée directement ou indirectement.

7. Utilisation selon le préambule de la revendication 5, **caractérisée en ce qu'**au moyen de la conductivité électrique mesurée et/ou la variation de la conductivité électrique et/ou de la permittivité diélectrique mesurée et/ou la variation de la permittivité diélectrique, des mesures d'entretien préventives sont indiquées longtemps avant des réfections préventives.

8. Utilisation selon le préambule de la revendication 5, **caractérisée en ce qu'**au moyen de la permittivité diélectrique et/ou la variation de la permittivité diélectrique, le contenu en eau des huiles est mesuré, et/ou au moyen de la variation de la permittivité diélectrique et/ou la variation de la conductivité électrique, la décomposition d'additifs est mesurée.

**9.** Utilisation selon le préambule de la revendication 5, **caractérisée en ce que** le volume entre les plaques d'électrode du capteur de base est la couche lubrifiante dans un palier à surveiller ou la couche lubrifiante entre les flancs de dents de roues dentées.

**10.** Utilisation selon la revendication 9, **caractérisée en ce qu'**additionnellement ou alternativement, l'épaisseur de la couche lubrifiante est mesurée.

**11.** Procédé selon une des revendications 1 à 3, **caractérisé par** une compensation de température adaptive à auto-apprentissage.

**12.** Procédé selon une des revendications 1 à 3, **caractérisé en ce que** les signaux mesurés sont transmis par l'intermédiaire d'une interface série, un système de bus et/ou une interface LAN ou WLAN de préférence à un système de surveillance basé sur le www.

**13.** Procédé selon une des revendications 1 à 3, **caractérisé par** un système de surveillance basé sur le www.

**14.** Utilisation selon le préambule de la revendication 5, **caractérisée en ce que** le système de capteur pour un mesurage direct de l'humidité est aussi utilisé pour des gaz agressifs et/ou corrosifs.

**15.** Utilisation selon le préambule de la revendication 5, **caractérisée par** l'utilisation comme capteur de carburant pour des véhicules et/ou machines à polycarburants.

**16.** Utilisation selon le préambule de la revendication 5, **caractérisée par** l'utilisation comme capteur de pétrole pour le mesurage de paramètres de pétroles et de sous-produits de ceux-ci.

**17.** Utilisation selon le préambule de la revendication 5, **caractérisée par** le mesurage d'ingrédients du pétrole, comme p.ex. des contaminations mécaniques, sels, acides, eau, savons d'huile se formant, etc. au moyen de mesurages de la conductivité électrique et de la permittivité diélectrique du pétrole.

Bild 1

Bild 2

Bild 3

Bild 4

Bild 5

Bild 6

*Bild 7*

Bild 8

Bild 9

*Bild 10.*

*Bild 11*

*Bild 12*

*Bild 13*

Bild 14

Bild 15

*Bild 16*

Bild 18

Bild 17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19628690
- DE 10217383
- DE 19963204
- DE 19914658